# EUROPEAN PATENT APPLICATION

(11) **EP 3 841 970 A1**
(43) Date of publication of application: **30.06.2021**
(21) Application number: 19219237.5
(22) Date of filing: 23.12.2019
(51) Int. Cl.: A61B 5/0408, A61B 5/00

(54) **DRY ELECTRODE FOR CONTACTING A SKIN OF A USER TO RECEIVE AND/OR TRANSMIT AN ELECTRICAL SIGNAL**

(71) Applicant: Koninklijke Philips N.V., 5656 AG Eindhoven (NL)
(72) Inventor: HILGERS, Achim Rudolf, 5656 AE Eindhoven (NL)
(74) Representative: Philips Intellectual Property & Standards

(57) **Abstract**

The present invention relates to a dry pin electrode (10) for contacting a skin (14) of a user (12) to receive and/or transmit an electrical signal. The electrode (10) comprises a base body (26) and a plurality of pins (30) protruding from the base body (26). Each pin (30) comprises a rubber, silicone and/or solid-gel material, and/or a retractable mechanism (42) or a spring-actuated mechanism (54); and/or an electroactive polymer.

## Description

### FIELD OF THE INVENTION

The present invention relates to a dry pin electrode for contacting a skin of a user to receive and/or transmit an electrical signal. The present invention further relates to an electroencephalographic system that comprises at least one of said dry pin electrode.

### BACKGROUND OF THE INVENTION

The present invention relates to an electrode for contacting a skin of a user to receive and/or transmit an electrical signal in order to measure electrical brain activity, also known as electroencephalography (EEG) or the measurement of an electroencephalogram. Electrical brain activity or EEG is measured by means of EEG electrodes contacting a skin of the user, in particular the scalp of the user.

One type of known electrodes is a wet or gel type electrode. The main challenge when applying wet or gel electrodes is to get a good, thus low, contact impedance to the skin. In clinical measurements, this is normally done with a (shower-cap like) rubber cap with integrated electrodes. These electrodes may include conductive rubber, graphite or metal.

The skin underneath these electrodes usually needs to be prepared by decreasing and often additional abrasion (e.g. removal of the dry top layer of the skin, the stratum corneum). A conductive gel is then applied between each electrode and the scalp (typically through a hole in the electrode or cap). This assures a low ohmic contact to the deeper skin layer (the epidermis) and "conversion" from ion current in the body to electron current in the EEG measuring system.

Using conductive gel also solves (partly) the problem of a varying distance between the electrode and the skin due to the variation from person to person with respect to a hair layer thickness and a hair density as well as temporal changes of distance that might occur due to motions of the head and/or body of the user.

However, in a lot of other cases not involving clinical measurements, for example, for lifestyle consumer products, for disabled patients or remote monitoring purposes, these kind of wet or gel electrodes are not practical. Furthermore, for example, if the cap is put over a users head, the wet electrodes might tip over and thus, will either have no electrical contact or need to be repositioned manually by an examiner. Hence, wet or gel electrodes might require time consuming and non-user friendly attachment procedures. Further, wet or gel electrodes might also require a regular re-fill or replacement due to drying out over time.

In order to cope with the aforementioned difficulties, dry electrodes for EEG measurements can be used as an alternative. These dry electrodes are usually designed, depending on their designated application place (forehead or occipital), as flat electrodes or pin electrodes. A dry flat electrode made of a conductive silicone material is, for example, shown in WO 2016/189422 A1.

For a dry electrode application on hairy skin areas of a user, the so called dry pin electrodes are used. These dry pin electrodes usually comprise a plurality of pins protruding from a base body of the electrode. Each pin has a contacting portion for contacting a preferably hair-free skin area on the scalp of the user. By means of the pins, the hair is preferably pushed away when the electrode is applied to the user's head.

It is known that a performance of the dry pin electrodes is dependent on the hair of the user, more precisely on the hair density and/or length. For a high hair density, in particular curly and/or very short hair, it might be difficult to reach the skin via the plurality of pins of the dry electrode, since high pushing forces need to be applied in order to get the contacting portion of the pin in good contact with the scalp of the user. For example, if the dry electrode is too elastic (soft), it might bend (move) away from the skin. In such cases either fine and thin stub based designs or a high(er) pressure needs to be used to maintain a good contact to the scalp. Both solutions may result in (additional) discomfort for the user. Furthermore, it is known that one source of noise (in the EEG measurements) is originated from movements of the dry electrode on the skin, i.e. caused by a head motion of the user. These noises are called "motion artifacts" causing EEG signal impurities.

In the Internal Committee for Biomedical Experiments study "PJ-011709 Dry electrodes for EEG" (ICBE-2-23648), different state of the art dry flat and pin electrodes have been evaluated. As a result, it has been found that none of the evaluated dry electrode performed ideally in all application cases, i.e. under all evaluation conditions (at all participants) and at all positions (forehead and occipital). For example, for an application on hairless skin, such as the forehead or any similar body location (e.g. for ECG or bio-impedance wearables), some state of the art dry flat electrodes performed well. However, also some state of the art pin (stub) electrodes showed good performances when applying them on a hairless skin portion of the user. With regard to the performance of dry electrodes on the hairy scalp, it was shown that dry flat electrodes are not suitable. However, some state of the art pin electrodes showed acceptable performances and were capable of measuring EEG signals within a predetermined tolerance. These pinned electrodes having such an acceptable performance usually comprise a coating layer on their contacting portions, such as a rigid AgCl (silver chloride) coating in order to improve the ion-electron conversion efficiency. The Ag/AgCl coating can be applied at a contacting part of the electrode being in contact with the skin. The coating causes conversion of ion currents into electron currents which are conductible by an electrically conducting base material of the dry electrode (which may be any electrically conducting material). The rigidity of the coating results in discomfort for the user, may have lifetime issues and does not stick to the skin. Thus, even though these dry pin electrodes have acceptable performances with respect to measurement results, they cause remarkable discomfort (red marks and pain) to the user and do not fulfill all requirements to be used, i.e., for long term EEG measurements.

Summarizing the aforementioned, the advantages of dry electrodes are accompanied by disadvantages, since dry electrodes might show drawbacks such as a user-discomfort, an increased skin-electrode impedance and higher sensitivity against motion artefacts. Also, when a dry electrode is applied to a patient, there might be difficulties getting sufficient signal quality due to the fact that the dry pin electrode might not be in good contact with the scalp under all circumstances.

### SUMMARY OF THE INVENTION

It is an object to provide a dry pin electrode that minimizes discomfort for a user, when the dry electrode is applied to the skin of the user, and shows an optimized (lifetime) and measurement performance.

According to a first aspect of the present invention, it is presented a dry pin electrode for contacting a skin of a user to receive and/or transmit an electrical signal, wherein the dry pin electrode comprises a base body and a plurality of pins protruding from the base body, wherein each pin comprises a rubber, silicone or solid-gel material, and/or a retractable or spring-actuated mechanism and/or an electroactive polymer. Furthermore, the present invention relates to an electroencephalographic system comprising at least one dry pin electrode according to the present invention.

According to a second aspect of the present invention, it is presented a system that comprises at least one of said dry pin electrode.

The present invention is based on the idea to overcome the problems and disadvantages of state of the art dry pin electrodes by proposing an advanced dry electrode having a plurality of pins each of which comprises a rubber, silicone and/or solid-gel material, a retractable or spring-actuated mechanism, an electroactive polymer or any combination thereof. Thus, the basic idea is to replace the state of the art AgCl coating of the contacting portions of the dry pin electrode by a soft, preferably sticky, and/or especially designed/shaped pin/contacting portion design.

The object is solved by manipulating the material and/or material composition of the pins of the dry electrode and/or the shape/design of the pins.

To guarantee a proper comfort for the user, each pin preferably has a thickness of at least 0.5 mm. The maximum length of each pin (measured from the base body end to the free front end (end face end)) is preferably not longer than approximately 5 mm, since a longer pin may start to bend. However, by manipulating the stiffness of the pins (i.e., with different materials), the pins may be designed longer. The electrode manufacturing process may include dip procedures (e.g. a droplet shape), additive manufacturing like 3D-printing or any suitable (mass) manufacturing technique. The electrode (i.e. the plurality of pins and the base body) is made of an electrically well conducting material.

In the case where the pin comprises the retractable or spring-actuated mechanism, an advantage is that the pinned dry electrode can be made of commercially available conductive silicones/rubbers with or without an AgCl coating, and at the same time showing a better performance, a more comfortable application and an improved skin adhesion.

The term "base body" defines a plate-like part of the dry electrode from which the plurality of pins protrudes.

The term "plurality of pins" means that the dry electrode comprises two or more pins. Each pin preferably protrudes from the base body at an angle between 70° and 100°, preferably 90°, respectively.

The term "electro active polymer (EAP)" names a class of materials within the field of responsive materials. Electroactive polymers are flexible and can generate high forces. Thereby, an EAP changes its mechanical form by applying a (drive- or control-) voltage (or drive current). This makes it possible to design EPA in such a way that they bend while applying said control voltage/current to them.

As mechanical bending has an effect like combing the hair of the user (i.e. pushing the hair away), the contact to the skin can be optimized. Furthermore, electro active polymers can work as sensors or actuators and can be manufactured such that they may adapt to various shapes and may be integratable into various systems and/or devices. Thereby, EAPs show good material characteristics, such as actuation stress and strain. Further advantages of EPAs are a low power requirement, a small form factor, a high flexible, a noiseless actuation, a high accuracy, a high resolution, a fast response and a reversible actuation.

Generally, it may also be possible to use only one EAP pin instead of a plurality of EPA pins. However, using a plurality of EAP pins has the advantage that each EAP pin can be designed such that it can bend in a differing direction with respect to the other pins. Thus, a chance of reaching the hairy scalp is improved.

According to a refinement of the present invention, each pin comprises a stub portion and a contacting portion arranged at an end face-end of the respective stub portion and configured to contact the skin of the user.

This should clarify that the contacting portion is, in other words, the tip of each bar- or stub shaped pin. The contacting portion may be designed as an individual component or it may be designed together with the stub portion as one piece, namely as the end face end of the stub portion.

According to a further refinement, the stub (or bar-shaped) portion of the pin preferably has a length extension that is longer than its width extension. The contacting portion is preferably in direct contact with skin (i.e. scalp) of the user when the dry electrode is applied to the user's scalp. Advantageously, each contacting portion can be slightly enlarged with respect the respective stub portion, i.e. such that a diameter of the contacting portion is larger than a diameter of the respective stub portion.

Especially in the case where each pin comprises a rubber, silicone and/or solid-gel material, it is preferable that the contacting portion of each pin is covered/coated with a soft coating comprising rubber, silicone and/or solid gel or that the whole contacting portion is made of at least one of these materials.

In order to realize a proper comfort for the user, said coating preferably has a predefined softness. Typically, said tissue/skin has a shore OO value between 10 and 30. Therefore, the rubber, silicone and/or solid gel coating (or any additional coating applied to the contacting portion of the pin) should have a similar shore value.

According to another refinement of the present invention, the contacting portion of each pin is detachably connected to the end face-end of the stub portion of the respective pin.

This refinement has the advantage that the pin does not need to be coated with rubber, silicone and/or solid-gel material, since the contacting portion can be made of at least one of the rubber, silicone or solid-gel material as a whole. Thus, the contacting portion can be mounted on the end face end (tip) of the stub portion of each pin.

This has the advantage that the contacting portion is replaceable when its respective lifetime is over or once its proper function is not guaranteed anymore. Thus, not the whole electrode needs to be replaced, but only the contacting portions of the pins.

The aforementioned refinement is especially preferable when the pin comprises the rubber, silicone and/or solid-gel material and/or when each pin comprises the retractable or spring actuated mechanism.

According to another refinement, the contacting portion is pivotally connected to its respective stub portion via a pivot element.

This refinement has the advantage that a higher degree of freedom for each pin is provided by means of the pivotable connection. The pivot element may be considered as a joint connection (e.g. in the form of a ball joint).

Preferably, the pivotable connection is designed as a solid state joint, in which flexibility/mobility is achieved by a weakened material area, e.g. in the form of a groove. Since the connection between each (preferably replaceable) contacting portion and each stub portion of each pin can be realized as a pivot element allowing an independent tilting of each contacting portion, the contractibility between the contacting portion and the skin (i.e. scalp) can be improved. This results in lower electrical impedance between the skin and the electrode. Furthermore, such a configuration is advantageous, since natural curvatures, i.e. of the scalp, in a contacting area of each electrode can be compensated.

According to another refinement of the present invention, the contacting portion has a shore OO value in a range of 50 to 80.

According to this refinement, it is preferable that an electrically conducting silicone or rubber may be used as a base material for the plurality of pins and/or the base body.

Too soft pins would bend while penetrating the hairs and thus would not get in (good) electrical contact with the scalp. Too stiff pins would cause discomfort to the patient. Thus, a shore OO value (stiffness) between 60 and 80 is preferable.

The shore value is preferably chosen depending on the patient's hair (length, density and/or shape, etc.).

According to another refinement, each pin comprises an adhesive cover layer covering the contacting portion and/or the stub portion.

Such an additional adhesion mechanism for improved adhesion to the skin of the user improves proneness against motion artefacts of the electrode. Furthermore, this refinement has the advantage that the adhesive effect of the dry electrode can be improved based on the adhesive cover layer. Preferably, said cover layer is coated onto the contacting portion and/or the stub portion of each pin. Preferred adhesive cover layers are soft and adhesive silicones (a potential supplier may be the Technogel GmbH). This refinement is especially preferable when each pin comprises the rubber, silicone and/or solid-gel material and/or the retractable mechanism or the spring-actuated mechanism.

According to another refinement, the contacting portion comprises a cup-shaped suction cup.

This refinement improves in particular the adhesion (stickiness to the skin of the user) by manipulating the shape of the contacting portion (soft tip add-on) of each pin. Instead of a rather flat, round or droplet-shaped contacting portion, the design of each contacting portion is based on (or inspired by) a suction cup, i.e. a suction bottom used for fixing on planar surfaces (such as tiles). Such a suction cup uses negative fluid pressure of air to adhere to nonporous surfaces, creating a partial vacuum when being applied to a surface. The refinement is especially preferable when each contacting portion comprises a rubber, silicone and/or solid-gel material and/or when each pin comprises the retractable or spring actuated mechanism.

According to another refinement, the suction cup has a cavity facing towards the skin of the user, when the dry electrode is applied to the skin of the user.

The material of the suction cup (suction cup-shaped contacting portion) is preferably soft, such that sealing between each cup-shaped contacting portion and the users skin is realized. Preferably, the suction cup is designed such that no (or only little) air is flowing back inside the cavity after applying the dry electrode to the skin. Thus, adhesion of the electrode can be improved.

According to another refinement, the retractable mechanism is configured to retract from a folded state to an unfolded state, when a predetermined pressure is applied thereto.

Such a refinement has the advantage that motion artefacts can be compensated by means of the retractable mechanism. In particular, (rather) constant contact impedance towards the skin of the user is guaranteed, which typically depends on the applied pressure with which the electrode is pushed onto the skin of the user. Said retractable mechanism may be realized such that each contacting portion or each pin is designed similar to a retractable (silicone) cup.

The retractable/foldable mechanism preferably comprises one or more (joint connections (such as solid state joints). By means of the joint connections, the retractable mechanism can be folded and unfolded. To bring the retractable mechanism in the folded state, the predetermined pressure has to be applied thereto. When a lower pressure than the predetermined pressure is applied, the retractable mechanism preferably behaves in the manner of an elastic spring.

With this, a better user comfort is realized. The idea is that the retractably designed pin changes its length when the predefined pressure/force is applied thereto, i.e. pressing the electrode against the scalp. Depending on the specific design and material properties, the compressed, retractable mechanism can retract/unfold (automatically) if the predetermined pressure/force is released again, i.e. by pulling of an electrode head net. To support said preferably self-retracting functionality, each pin may be designed as a closed cavity. If the length gets shortened by pressure/force application, air inside the closed cavity will be compressed. Accordingly, a resulting overpressure may support the self-retracting ability.

Preferably, an above-mentioned (adhesive) coating or separate add-on (i.e. that improves electrical conductibility) may be added to an end face end of each contacting portion in order to improve the contact to the skin of the user.

According to another refinement, each spring-actuated mechanism comprises a spring element which is arranged between the base body and the respective contacting portion.

This refinement has the advantage that by means of the spring-actuated mechanism, the user convenience is improved and a proper (electrical) connection between the contacting portions and the users head (scalp/skin) is guaranteed. For example, each pin or each stub portion may comprise two parts which can be moved relative to one another. Between these to movable parts, the spring element may be arranged to initiate a length change via an applied spring force. Thus, the length of each pin may be adjusted individually when applying the dry electrode to the user. If a pressure/force gets too high, each spring may be contracted causing shortening of each pin, which avoids inconvenience/pain for the user. It may be preferable that the spring element is integrated in the stub portion.

Furthermore, this refinement shows an advantage in view of WO 2013/038285 A1, in which a single spring-actuated mechanism is only arranged at a pedestal of a dry electrode. In contrast to the state of the art, with the dry pin electrode according to this refinement, irregularities on the scalp can be smoothed out more easily. Thus, with this refinement, the flexibility and adaptability is improved with respect to the state of the art.

According to another refinement, the spring-actuated mechanism is a mechanical, electro-mechanical and/or pneumatic spring mechanism.

Instead of a purely mechanical spring-actuated mechanism, a pneumatically actuated mechanism may be used for pushing, for example, each pin with its contacting portion towards the scalp of the user. Each pin can be pulled back, i.e. by creating an underpressure within each stub section. Furthermore, it may be convenient to use an electromechanically actuated spring mechanism, pushing each pin with its contacting portion towards the head and pulling it back, respectively. This could be realized by micro-electronic linear motors or piezo actuators.

According to another refinement, the spring mechanism is controlled based on an impedance value measured between the contacting portion and the skin of the user.

In this refinement, an electronic control mechanism (control loop) is used to optimize the pressure of each pin towards the scalp of the user improving connectivity and lowering the risk of motion artifacts. Thereby, for example, elasticity as well as a length contraction of each pin may be controlled for each pin separately.

According to another refinement, each pin is coated on its respective outer surface with an electrically conductive silicone.

In this refinement, the stub portion and the contacting portion are preferably designed as one piece which is made of an electroactive polymer. The electroactive polymer preferably has a pin shape and is covered/coated with an electrically conducting silicone. The shore OO value of the silicone is preferably adapted to a respective shore value of the skin of the user.

According to another refinement of the present invention, the electroactive polymer includes at least one of a dielectric elastomer, a polyvinylidenfluorid based relaxor ferroelectric polymer, a liquid crystal elastomer, a conjugated polymer, a carbon nanotube polymer or an ionic polymer metal composite.

Generally, EAPs can be subdivided in field driven and ionic driven materials. Examples of field driven EAPs are electrostrictive polymers such as dielectric elastomers, polyvinylidenfluorid (PVDF) based relaxor ferroelectric polymers and liquid crystal elastomers (LCE). Examples of ionic driven EAPs are conjugated polymers, carbon nanotube (CNT), polymer composites and ionic polymer metal composites (IPMC). Field driven EAP's are actuated by an electric field through direct electromechanical coupling, while an actuation mechanism for ionic EAPs involves diffusion of ions and/or electrochemical oxidation and reduction.

According to another refinement, each pin comprises a tilting mechanism which is configured to tilt the pin with respect to the base body.

One advantage of this refinement is that due to the tilting mechanism, each pin is individually pivotable with respect to the base body. This is different with respect to the state of the art. For example, WO 2013/038285 A1 shows an electrode having one general tilting mechanism for tilting the base body of the electrode, but not a tilting mechanism for each individual pin.

However, tilting of each individual pin has the advantage that natural contours of the scalp of the user can be compensated by means of tilting each individual pin in a slightly different directing with respect to one another. The tilting of each pin is preferably done by means of a joint connection (e.g. in the form of a ball joint) or by a solid state joint connection, in which flexibility/mobility is achieved by a weakened material area, e.g. in the form of a (annular) groove in base body side end of each stub portion.

### BRIEF DESCRIPTION OF THE DRAWINGS

These and other aspects of the invention will be apparent from and elucidated with reference to the embodiment(s) described hereinafter. In the following drawings
Fig. 1 shows an application case of a first embodiment of a dry electrode;
Fig. 2 shows the first embodiment of the dry electrode in a perspective view;
Fig. 3 shows a second embodiment of a dry electrode in a perspective view;
Figs. 4A, 4B show a third embodiment of a dry electrode having retractable pins;
Figs. 5A, 5B show a fourth embodiment of a dry electrode having pins that comprise an electroactive polymer;
Figs. 6A, 6B show a fifth and a sixth embodiment of a dry electrode, which pins comprise a spring-actuated mechanism; and
Fig. 7 shows a seventh embodiment of a dry electrode having pins with controllable spring-actuated mechanisms.

### DETAILED DESCRIPTION OF THE INVENTION

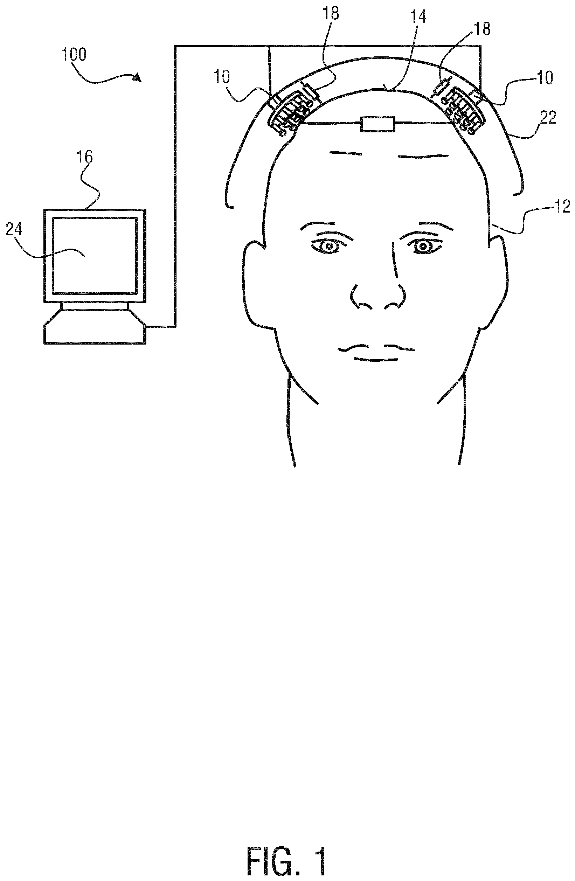

Fig. 1 shows an exemplary use case of an EEG system. The EEG system is denoted in its entirety with reference number 100. The EEG system 100 comprises a plurality of dry electrodes 10, wherein in Fig. 1 two electrodes 10 are shown. The dry electrodes 10 are designed as dry pin electrodes.

Brain waves of the user 12 are typically sensed by means of two, or more, EEG electrodes 10 being in electrical contact with a skin 14 (i.e. scalp) of the user 12 (e.g. person). The two EEG electrodes 10 of Fig. 1 are wired to an EEG of electronic circuitry, which may be included in a processing unit 16. The processing unit 16 is connected to both electrodes 10 via one or more cables.

The EEG processing unit 16 is represented by a computer, but may also be a microprocessor (µP) or any other suitable processing device.

To obtain sufficient EEG signal quality using the dry electrodes 10, a respective contact impedance 18 between each electrode and the users scalp 14 should stay below a certain threshold (e.g. between 10-100 kQ, preferably 50 kQ,), depending on the EEG phenomena. The contact impedance depends on the input impedance and a design of the used amplifier (i.e. which impedances the amplifiers "allow" for proper signal processing). For higher contact impedances (i.e. in the order of ≤ 1 MΩ), the picked up brain signals can be dominated by noise, which may no longer be filtered out. Furthermore, it should be noted that in order to receive proper signal amplification, the contact impedance 18 has to be balanced between the electrodes 10. In order to keep the contact impedance 18 of each electrode 10 under the threshold, a proper contact between the respective electrode 10 and the scalp 14 of the user 12 is preferable.

The electronic circuit is closed by a current flow between the two electrodes 10. Thereby, the brain as well as the skull of the user 12 comprises brain impedances 20, which is connected in parallel to the contact impedances 18. However, since the brain impedance 20 is several orders of magnitudes lower than the contact impedances 18, it is neglectable. Thus, ion current in the brain can be transformed to electronic current.

Generally, impedance measurements are actively realized by means of electronics. Thereby, most of the current between (i.e. two or more) electrodes does not flow through the brain, since the current would primarily need to flow through the skull which has a high impedance value. Accordingly, the current flows through a tissue underneath the scalp 14 of the user 12. In case of EEG measurements, a potential difference between at least two electrodes is measured, wherein only a small ionic current reaches the electrodes 10. This is the reason why the contact impedance should be as low as anyhow possible. In Fig. 1, the electrodes 10 are arranged at an EEG head net or cap 22. A measured and processed EEG signal may be represented on a display 24.

In other embodiments, the processing unit 16 may be connected wirelessly (i.e. via Bluetooth or WLAN) to the electrodes 10. Furthermore, the electrodes 10 do not need to be arranged at an EEG cap 22, but may also be connected separately to the user's scalp 14. In other embodiments, further electrodes (such as flat dry electrodes) may be arranged at the user's forehead and/or hairs in order to improve the EEG signal quality.

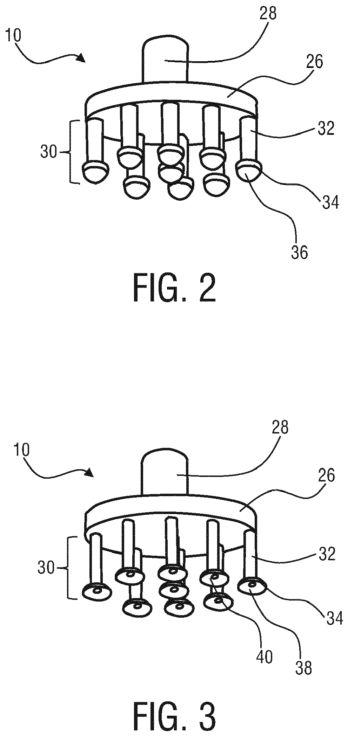

Fig. 2 shows a first embodiment of an electrode 10, which is designed as a dry pin electrode 10. The dry pin electrode 10 comprises a base body 26. The base body 26 may be designed as a flat disk or plate-like part having two opposing, preferably flat faces. A pedestal 28 protrudes from one of the two faces. The pedestal 28 may be used, as a cable guide, i.e., for connecting the electrode via cables to the processing unit 16 and for arranging (fixing) the electrode 10 on the EEG cap 22. A plurality of pins 30 protrude from the opposing face of the base body 26, namely preferably in an opposite (but parallel) direction to the pedestal 28.

Each pin 30 comprises a stub portion 32 and a contacting portion 34 configured to contact the skin 14 of the user 12. Each contacting portion 34 is arranged at an end face-end of each stub portion 32 and may be connected fixedly or detachably to the stub portion 32.

The stub portion 32 may be designed with a hole at its end face end in which the contacting portion 34 can be fitted (or vice versa). In Fig. 2, each contacting portion 34 is detachably connected to its respective stub portion 32, e.g. via a clamping connection or a micro screw connection (not shown). Each contacting portion 34 is shaped as a hemisphere having a diameter that is larger than a diameter of the respective stub portion 32.

Each contacting portion 34 is made of an electrically conducting material, such as rubber, silicone, solid gel, hydrogels or any combination thereof. In the case of Fig. 2, each contacting portion may be covered/coated with an adhesive layer 36.

It is preferable to use a material that has an intrinsically and efficient ion-electron conversion mechanism. An example for a soft conducting silicone/rubber is described in WO 2016/189422 A1, where an efficient ion-electron conversion mechanism is realized by mixing detergents into a conductive silicone (e.g. using as a base/raw material SILPURAN® 2400/25 A/B from Wacker having an intrinsically shore OO value of 25). As a proper alternative, solid-gels like SolidGeltrode from Neuroelectrics ([Online], https://www.neuroelectrics.com/products/electrodes/solid-gel-consumable/) may be used. As an alternative, a solid gel as described in (e.g. M. I. e. al, "Cholinium-based ion gels as solid electrolytes for long-term cutaneous electrophysiology," Journal of Materials Chemistry C, pp. 8942-8948, 2015) can be used as a contacting portion material.

Since the afore-mentioned materials are soft (having a low shore value), wearing comfort for the user 12 is improved and at the same time prone to motion artefacts is minimized, since these materials comprise an intrinsic stickiness (the softer the material, the stickier it is). It should be noted that the afore-mentioned materials may be particularly suitable for the contacting portions 34, since their shore value might be too low to use them as a base material, for example, for the stub portions 32 and/or the base body 26. The issue thereby is that due to the low shore value, stub portions 32 made of these materials would tend to bend if the stub portions 32 have been too long, while applying them through the hair and thus, they hardly would reach the hairy scalp 14 of the user 12. The stub portion 32 and the base body 26 are therefore preferably made of comparatively harder materials.

Fig. 3 shows a second embodiment of the dry electrode 10. Therein, each contacting portion 34 is designed as a suction cup 38, similar to a small suction button. The stub portions 32 of Fig. 3 have a smaller diameter than the stub portions 32 in Fig. 3. Each of the suction cups 38 has a respective cavity 40 that faces towards the skin 14 of the user 12, when the dry electrode 10 is applied to the skin 14 of the user 12. While applying these suction cups 38 to the skin 14 of the user 12, air accumulating between the skin 14 and the respective cavity 40 of each suction cup 38 is squeezed out causing an underpressure inside each cavity 40.

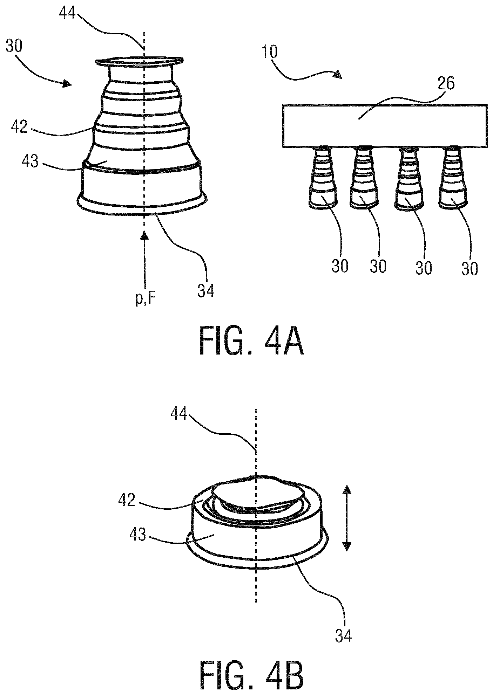

Figs. 4A and 4B show a third embodiment of the dry electrode 10. In this embodiment, each pin 30 comprises a retractable mechanism 42. The retractable mechanism 42 of each pin 30 is provided such that each pin 30 is designed as a retractable cup 43.

Fig. 4A shows the retractable cups 43 before applying the electrode 10 to the user 10. The retractable cups (or retractable pins) 43 are in a decompressed state, in which the "retractable cups" are unfolded. The retractable cups 43 are preferably designed as a closed cavity that supports the self-retractability. The retractable mechanism 42 is configured to retract the pin 30 from a folded state to an unfolded state or vice versa when a predetermined pressure is applied thereto.

Fig. 4B shows the retractable pins 30 after or while applying the electrode 10 to the user's head. While applying the pinned electrode to the user 12, a predefined pressure p and force F is applied to the electrode 10, i.e. by strapping the EEG cap 22 with the electrodes 10 over the head of the user 12. This predefined pressure p and force F causes a compression of each of the retractable pins 30 in a respective longitudinal direction 44. The higher the pressure, the shorter the retractable pins 30 will get. Depending on the design of the retractable pins 30, this does not need to be a linear correlation, but any other non-linear one. I.e. at a low applied pressure p the change in length change is lower than when a higher pressure(s) is applied (or vice versa).

The retractable pins 30 behave like a mechanical spring, (automatically) adjusting their respective length depending on the applied pressure p and force F. Alternatively, the pins 30 may be designed in such a manner that if applying a predefined pressure, their length becomes shorter, while their diameter/width becomes larger.

In yet another embodiment, the closed retractable conductive silicone pin 43 is connected to and operated by air. If an EEG head net/cap 22 is applied to the user's head, an overpressure is generated inside the closed cavity of each retractable cup 43. By means of adjusting said overpressure, the contact pressure between skin 14 and the electrode 10 (or the plurality of contacting portions 34 can be controlled. If the pin 30 needs to be shortened, the overpressure may be reduced (a vacuum may be generated). Furthermore, a pressure sensor may be attached to the contacting portion 34 of each retractable pin 43, said pressure sensor being configured to control a respective contact pressure between the skin 14 and each contacting portion 34.

Generally, it should be mentioned that other arrangements of the retractable mechanism 42 (like upside down and interchanging ones) are possible

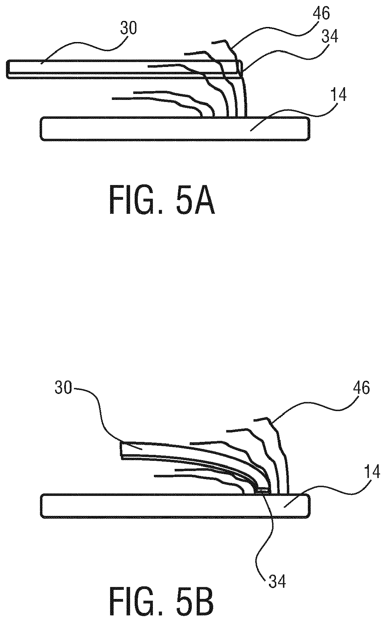

Figs. 5A and 5B show a fourth embodiment of the dry pin electrode 10. Therein, each pin 30 is made of an electroactive polymer (EAP) which is preferably coated with an electrically conductive silicone layer. Thus, the base of each pin 30 is preferably made of an EAP material and coated with a silicone layer. A specialty of EAP materials is an electrical actuation while electromechanical measurements may be performed at the same time (as described in EP 3345227B1). Accordingly, such functionality may be used to identify and optimize the most convenient and/or efficient contact pressure.

While applying the EEG head net 22 to the users head, the EAP pin 30 is not electrically activated and thus, shows an essentially flat, straight bar-like shape (see Fig. 5A). Once the head net 22 is positioned on the users head, a driving voltage and/or drive current is applied to the EAP pins 30, causing the EAP pins 30 to mechanically deform (or bend) until the contacting portion 34 (namely the tip of each bar-like pin 30) of the preferably silicone-coated pin 30 reaches the hairy scalp 14. A contact position may be controlled by measuring the impedance between two (neighbored) EEG electrodes 10. Furthermore, vibrating activation of the EAP pins 30 may improve penetration through hair 46 of the user 12. Due to bending of each EAP pin, the hair 46 is pushed away causing an improved connection between each contacting portion and the scalp 14.

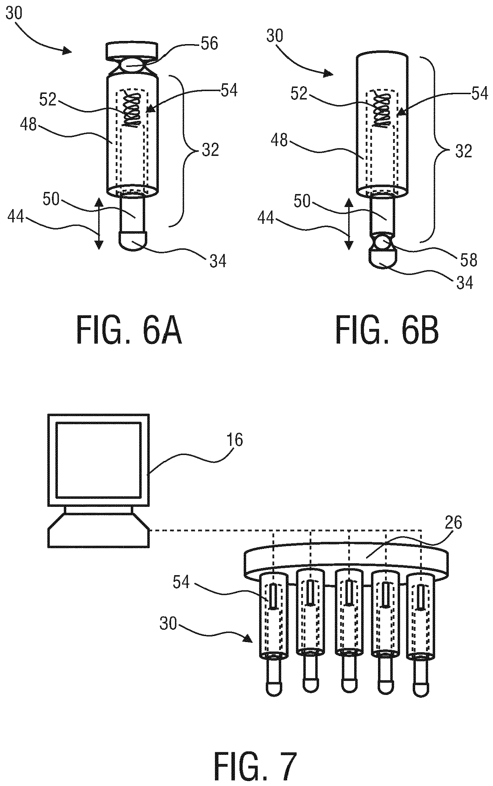

Figs. 6A and 6B show a fifth and sixth embodiment of the pin 30 of a dry electrode 10. In Fig. 6A, the stub portion 32 of the pin 30 comprises a first part 48 and a second part 50. The first part 48 is movably connected to the second part 50 via a spring element 52 (i.e. a spiral spring). Together, the first part 48, the second part 50 and the spring element form a spring mechanism 54. The first part 48 may be designed as a hollow cylinder, whereas the second part 50 may be designed as a bar, which is movable inside the hollow cylinder along the longitudinal direction 44 of the pin 30. Thus, a length of the pin can be manipulated via the spring element 52, namely by moving the second part 50 in and out of the first part 48.

Furthermore, the pin 30 of Fig. 6A comprises a tilting mechanism 56 arranged on the end face end of the stub portion 32, or better to say on the end face end of the first part 48 of the stub portion 32. The tilting mechanism 56 comprises a ball joint but may also be designed as a solid state joint. Due to this tilting mechanism 56, each pin 30 has at least one degree of freedom for tilting around its longitudinal direction 44. In contrast to Fig. 6A, in Fig. 6B, the contacting portion 34 of the pin 30 comprises a pivot element 58 causing that the contacting portion 34 is pivotable with respect the stub portion 32.

Fig. 7 shows a further embodiment of the dry electrode 10. In this embodiment, the spring mechanism 54 of each pin 30 is controlled, e.g. by means of the processing unit 16 (that may further comprise a control unit for such controlling).

In Fig. 7, the spring mechanism 54 is actuated pneumatically (indicated by small compression cylinders inside each stub portion 32). In order to control the spring mechanism 54, for example, the skin-electrode contact impedance 18 may be measured between at least two different electrodes 10 by means of the electronic circuit. As long as the skin-electrode impedance 18 is above a certain threshold (i.e. 50 kΩ), the control unit may be configured to control the spring mechanism 54 of each pin 30 such that it is pressed harder against the scalp 14 of the user 12, i.e. by stretching the compression cylinders. If more than two electrodes 10 are available, all possible skin-electrode impedance combinations may be measured to optimize the electrode pressure distribution control, especially for EEG nets 22 with a high electrode density (lots of electrodes). In other embodiments, the spring mechanism 54 may be electro-mechanically or pneumatically actuated by micro motors.

According to another embodiment (not shown), the pedestal 28 of the dry electrode 10 can be made of a softer conductive silicone than the base body 26. With this material configuration, a natural solid state joint is provided, allowing tilting between the pedestal 28 and the base body 26. Alternatively a pivot element or bending mechanism may be applied between the pedestal 28 and the base body 26. Such a mechanism allows that the electrode 10 (as a whole) fits better to a shape of the scalp 14 of the user 12.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Dry pin electrode for contacting a skin (14) of a user (12) to receive and/or transmit an electrical signal, the electrode (10) comprising a base body (26) and a plurality of pins (30) protruding from the base body (26), **characterized in that** each pin (30) comprises:
a) a rubber, silicone and/or solid-gel material; and/or
b) a retractable mechanism (42) or a spring-actuated mechanism (54); and/or
c) an electroactive polymer.

2. Dry pin electrode as claimed in claim 1, wherein each pin (30) comprises a stub portion (32) and a contacting portion (34) arranged at an end face-end of the respective stub portion (32) and configured to contact the skin (14) of the user (12).

3. Dry pin electrode as claimed in claim 2, wherein the contacting portion (34) is detachably connected to the end face-end of the stub portion (30).

4. Dry pin electrode as claimed in claim 2 or 3, wherein the contacting portion (34) is pivotally connected to its respective stub portion (32) via a pivot element (58).

5. Dry pin electrode as claimed in any of claims 2 to 4, wherein the contacting portion (34) has a shore OO value in a range of 60 to 80.

6. Dry pin electrode as claimed in claims any of 2 to 5, wherein each pin (30) comprises an adhesive cover layer (36) covering the contacting portion (34) and/or the stub portion (32).

7. Dry pin electrode as claimed in any of claims 2 to 6, wherein the contacting portion (34) comprises a cup-shaped suction cup (38).

8. Dry pin electrode as claimed in claim 7, wherein the suction cup (38) has a cavity (40) facing towards the skin (14) of the user (12), when the dry electrode (10) is applied to the skin (14) of the user (12).

9. Dry pin electrode as claimed in any of claims 1 to 8, wherein the retractable mechanism (42) is configured to retract from a folded state to an unfolded state, when a predetermined pressure is applied thereto.

10. Dry pin electrode as claimed in any of claims 2 to 8, wherein each spring-actuated mechanism (54) comprises a spring element (52) which is arranged between the base body (26) and the respective contacting portion (34).

11. Dry pin electrode as claimed in claim 10, wherein the spring-actuated mechanism (54) is a mechanical, electro-mechanical and/or pneumatic spring mechanism (54).

12. Dry pin electrode as claimed in claims 10 or 11, wherein the spring-actuated mechanism (54) is controlled based on an impedance value (18) measured between the contacting portion (34) and the skin (14) of the user (12).

13. Dry pin electrode as claimed in any of claims 1 to 12, wherein the electroactive polymer includes at least one of a dielectric elastomer, a polyvinylidenfluorid based relaxor ferroelectric polymer, a liquid crystal elastomer, a conjugated polymer, a carbon nanotube polymer or an ionic polymer metal composite.

14. Dry pin electrode as claimed in any of claims 1 to 13, wherein each pin (30) comprises a tilting mechanism (56) which is configured to tilt the pin (30) with respect to the base body (26).

15. An electroencephalographic system (100) comprising at least one dry pin electrode (10) according to any of claims 1 to 14.
